# EUROPEAN PATENT SPECIFICATION

(11) **EP 0 938 350 B1**
(45) Date of publication and mention of the grant of the patent: **29.11.2006**
(21) Application number: 97943686.2
(22) Date of filing: 09.10.1997
(51) Int. Cl.: A61L 27/00

(54) **BIOMEMBRANE SUITABLE FOR USE IN SUBSTITUTION, RECONSTRUCTION, INDUCTION OF ANGIOGENESIS, NEOFORMATION OR REGENERATION OF HUMAN OR ANIMAL ORGANS OR TISSUES**
BIOMEMBRAN ZUR SUBSTITUTION, REKONSTRUKTION, INDUKTION DER ANGIOGENESE, NEUBILDUNG ODER REGENERATION VON MENSCHLICHEN ODER TIERISCHEN ORGANEN ODER GEWEBE
BIOMEMBRANE DESTINEE A ETRE UTILISEE DANS LA SUBSTITUTION, LA RECONSTRUCTION, L'INDUCTION D'ANGIOGENESE, LA NEOFORMATION OU LA REGENERATION D'ORGANES OU DE TISSUS HUMAINS OU ANIMAUX

(30) Priority: 09.10.1996 BR 9604371
(43) Date of publication of application: 01.09.1999
(73) Proprietor: Da Silva Zborowski, Antonio César, S o José do rio Preto, SP (BR); Mrue, Fatima, Ribeir o Preto SP (BR)
(72) Inventor: Da Silva Zborowski, Antonio César, S o José do rio Preto, SP (BR); Mrue, Fatima, Ribeir o Preto SP (BR)
(74) Representative: Goddar, Heinz J.
(86) International application number: PCT/BR1997/000054
(87) International publication number: WO 1998/015300

(56) References cited:
- EP-A- 0 568 413
- EP-A- 0 621 013
- WO-A-89/06523
- DATABASE WPI Section Ch, Week 9641 Derwent Publications Ltd., London, GB; Class A96, AN 96-410726 XP002052985 & SU 1 251 528 A (BIOLOG PHYS INST) , 10 February 1996

## Description

### Field of Invention

The present invention relates to a biosynthetic biomembrane which can be used as substitute material and/or an inductor of neoformation of organs and tissues or as a support for the adhesion and growth of microorganisms, cells, and tissues as well as for the interaction of chemical and/or therapeutic purposes, "in vitro" and "in vivo",

### Background of the Invention

Up to this moment no material has been developed to be used in the human or animal bodies as inductor of neoformation of organs or tissues or to act as an stimulator of the angiogenesis of such tissues or organs acting as a support for the growth of microorganisms and new cells in living organisms. Most of the products known from the prior art are produced from synthetic polymeric material, such as polypropylene. These are inert materials and are used, for example, in the correction of defects of abdominal and diaphragm, as a substitute of cardiac valves, or even as wound dressings but without stimulating the angiogenesis and/or neoformation.

But the above known materials present some considerable disadvantages, namely the risk of infection and their low elasticity and, in some cases, their antigenicity since the materials are heterogenous.

An alternative for the replacement of some organs or parts thereof is to use biological material extracted form other organs. Illustrative examples are stomach and intestine which can be use to replace the esophagus and intestinal patches to replace the bladder. But this technique requires the use of complicated surgical procedures, performed in prolonged operative time which brings about a high level of morbidity and mortality. Moreover, some organs such as nerves, penis or trachea cannot be replaced by a different organ.

It is, therefore, an objective of the invention to provide a suitable material to be used as a substitute for tissues and organs, or to allow the formation of new tissues and organs which are substantively homogenous, present high elastic characteristics and do not show any considerable risk of infection.

### Summary of the Invention

The object of the invention is defined in the claims.

The present invention refers to a biomembrane suitable for use in substitution, reconstruction, induction of angiogenegis, neoformation or regeneration of human or animal organs or tissues which comprises the polymerization product of natural latex as its main constituent.

The invention is also related to a substitute material for human or animal organs and tissues which contains a biomembrane as described above.

In another aspect, the invention refers to a support for the growth of microorganisms and cells which is made from a biomembrane as described above.

### Detailed Description of the Invention

It has now been found that the polymerization product of natural latex provides excellent physical and chemical properties which render a suitable polymer material which can be successfully used primarily in the substitution, reconstruction or regeneration of a large number of organs or tissues of human or animal organisms. In one preferred embodiment of the invention, natural latex extracted from a rubber tree selected from *Hevea brasiliensis, Partheniun argentatum* (guayule), *Ficus elastica,* and the derived subspecies thereof, is used. Most preferably, the latex from *Hevea brasiliensis* is used.

The term "polymerization product" as used herein means the products obtained by the spontaneous polymerization of chemical compounds present in the naturally occurring latex, that is to say, the latex directly extracted form the rubber trees. The polymerization conditions may vary according with the specific kind of desired product and the associated characteristics necessary for each particular future application. Therefore, the polymerization process may be carried out in any usual and conventional way provided that it preserves the biological activity of the chemical constituents of the original latex, mainly the protein that stimulates angiogenesis.

One of the improved characteristics of the biomembrane of the present invention is that its resembling of surface with the cellular membranes makes it possible the cellular anchorage and the interaction of recognition among the biological surfaces, without operating the natural mechanisms of defense against the material, which would be harmful to the organism in question. The biomembrane according to the invention allows and stimulates the adhesion of microorganisms and/or cells establishing an adequate environment for the development and growing thereof which is very close to the physiological cellular interaction. It strongly stimulates the angiogenesis which along with the adhesion proteins such as collagen or polylysin optimize and organize the processes of tissular regeneration/neoformation.

Another important advantage of the present invention is its ability of accepting the introduction therein of chemical and/or biological substances of simple or complex structure, which is highly important specially in the cases in which the maintenance of conformational structure for biological activity is necessary. This ability makes it possible that for the biomembrane of the present invention to be used as a support for the interaction of chemical and/or therapeutic purposes. The biomembrane may be molded into simple or complex prosthesis, for example, making it a matchless material for the manufacturing or prosthesis of organs or tissues.

The biomembrane according to the invention will have, therefore, clinical, laboratory and industrial application. In the clinical ambit, this material is used for the purpose of the manufacturing of prosthesis for replacement of organs and tissues such as: esophagus, stomach, intestine, arteries, trachea, nerves, bones, skin, abdominal wall, diaphragm, bladder, penis, testicles and others, as well to induce the regeneration or neoformation thereof. It will serve, also, to help in the prevention of complications resulting from the use of vascular prosthesis and others, through the covering of such prosthesis with the biomembrane. As far as the laboratory and industrial aspects are concerned, the purpose of the biomembrane is to be the support for the growth or microorganisms, cells and tissues, and interaction of substances with "in vivo" and "in vitro" diagnostic and/or therapeutic objectives. Since the biomembrane structure easily allows the introduction of substances such as specific antibodies agaisnt certain diseases, it can be uses for example, in AIDS diagnosis. In this event, the biomembrane associated with a HIV antiviral sequence is allowed to react with the patient biological fluids by using fluorescence methods or binding reactions for the desired detection.

The clinical use of a product in accordance with the invention as basic material for manufacturing prosthesis, acting as an inductor of neoformation of organs and tissues provides a significant improvement of the quality of life for the patients whose organs like esophagus, intestine, bladder, penis, nerve, trachea, bone, skin, soft tissues of members and others, for any given reason, have been removed, injured or are not functioning properly.

The use of biomembrane as support for the growth of cells "in vivo" an/or "in vitro" allows the utilization of it as support for homologous or heterologous graft, for usage, for example, in the treatment of skin burning, in the plastic reconstruction after extensive skin drying up caused by injuries or aesthetic correction. As an inductor of granulation, it shortens, for example, the time required for healing ulcers of stasis, phlebopatic and bad irrigated organs, in addition to an increase in the speed of the process of healing of the abdominal wall in cases of peritoniostomy, strongly decreasing the time of patients hospitalization and reducing the complications originated from peritoniostomy.

It will be promptly recognized by anyone skilled in the art that the biomembrane of the invention may be used not only in the form of a molded article, such as a prosthesis, but it may also be formulated for other therapeutic applications, for example, in the form of a gel, a powder, a spray, or a foam, with or without the addition of chemical and/or biological substances.

The present invention will now be better illustrated by the following example which, however, just refers to a preferred embodiment thereof and should not be understood as limiting the general scope of the invention.

### Example

Natural latex extracted from rubber tree *Hevea brasiliensis* was treated with ammonium and centrifuged at 8.000 x g and filtered. Afterwards, hydroxide of potassium was introduced at a dose rate of 2,5 g/kg, letting the emulsion rest for approximately 30 minutes. After this time, a suspension of sulfur at 50%, 40 grams, per liter of latex was added with slow agitation, maintained and allowed to rest for 24 hours at room temperature. Then, formaldehyde with a pH between 7,0 - 8,5 was added, adjusted with sodium hydroxide in four sequential phases of 5.0 ml each, at 2 hour intervals, resulting in a total volume of 20 mL or until the ammonium to be neutralized. Next, polyvinyl-methyl-ether (20 ml per liter of latex) was added through slowly homogenization of the suspension during the entire process of the resin addition thus providing a biomembrane. The molding of the resulting biomembrane was accomplished by its polymerization over the surface with patterns specifically developed for the manufacturing of prosthesis or supports, followed by successive baths of the molding into the compound and a heating step carried out at a temperature lower than 80°C..

Then, polylysins at a concentration from O to 1% and from O to 2% of collagen were introduced and, finally, the macrobiomembrane was sterilized with oxide of ethylene.

## Claims

1. Use of natural latex for the manufacture of a polymerization product for the treatment of skin burning, in the plastic reconstruction after extensive skin drying up caused by injuries or aesthetic correction , ulcers of stasis, phlebopathie and bad irrigated organs or peritoniostomy.

2. Use according to claim 1, wherein the natural latex has been extracted from a rubber tree selected from the group comprising *Hevea brasiliensis, Parthenium argentatum* (guayule) and *Ficus elastica.*

3. Use according to any of claims 1 or 2, wherein the latex has been extracted from *Hevea brasiliensis.*

4. Use according to any of the preceding claims, wherein the latex endogenously comprises a protein that stimulates angiogenesis.

5. Use according to any of the preceding claims, wherein the polymerization product is a constituent of a biomembrane, a molded article, a gel, a powder, a spray or a foam.

6. Use according to any of the preceding claims, wherein the polymerization product comprises added chemical and/or biological substances.

7. Use according to any of the preceding claims, wherein the treatment is in an animal, preferably in a human.

## Patentansprüche

1. Verwendung von natürlichem Latex zur Herstellung eines Polymerisationsprodukts zur Behandlung einer Hautverbrennung, zur plastischen Rekonstruktion nach ausgedehnter Hautaustrocknung, verursacht durch Verletzungen oder durch eine ästhetische Korrektur, zur Behandlung von Ulcus cruris, phlebopathischen und schlecht durchbluteten Organen oder einer Peritoneotomie.

2. Verwendung nach Anspruch 1, wobei der natürliche Latex aus einem Kautschukbaum extrahiert worden ist, ausgewählt aus der Gruppe, die *Hevea brasiliensis, Parthenium argentatum* (guayule) und *Ficus elastica* umfasst.

3. Verwendung nach einem der Ansprüche 1 oder 2, wobei der Latex aus *Hevea brasiliensis* extrahiert worden ist.

4. Verwendung nach einem der vorangehenden Ansprüche, wobei der Latex endogen ein Protein umfasst, welches eine Angiogenese stimuliert.

5. Verwendung nach einem der vorangehenden Ansprüche, wobei das Polymerisationsprodukt ein Bestandteil einer Biomembran, eines geformten Artikels, eines Gels, eines Puders, eines Sprays oder eines Schaums ist.

6. Verwendung nach einem der vorangehenden Ansprüche, wobei das Polymerisationsprodukt zugesetzte chemische und/oder biologische Substanzen umfasst.

7. Verwendung nach einem der vorangehenden Ansprüche, wobei die Behandlung bei einem Tier, vorzugsweise bei einem Menschen erfolgt.

## Revendications

1. Utilisation de latex naturel pour fabriquer un produit de polymérisation destiné au traitement des brûlures cutanées, à la reconstruction plastique après une déshydratation cutanée étendue résultant de blessures ou de corrections esthétiques, au traitement des ulcères de stase et des organes présentant des phlébopathies et des défauts d'irrigation ou de péritonéostomies.

2. Utilisation selon la revendication 1, **caractérisée en ce que** le latex naturel a été extrait d'un arbre producteur de latex appartenant au groupe composé de *Hevea brasiliensis, Parthenium argentatum* (guayule) et *Ficus elastica.*

3. Utilisation selon la revendication 1 ou 2, **caractérisée en ce que** le latex a été extrait de *Hevea brasiliensis.*

4. Utilisation selon l'une des revendications précédentes, **caractérisée en ce que** le latex contient une protéine endogène stimulant l'angiogenèse.

5. Utilisation selon l'une des revendications précédentes, **caractérisée en ce que** le produit de polymérisation est un constituant d'une biomembrane, d'un article moulé, d'un gel, d'une poudre, d'un spray ou d'une mousse.

6. Utilisation selon l'une des revendications précédentes, **caractérisée en ce que** le produit de polymérisation contient des substances chimiques et/ou biologiques ajoutées.

7. Utilisation selon l'une des revendications précédentes, **caractérisée en ce que** le traitement est destiné à un animal, et de préférence à un sujet humain.
